# EUROPÄISCHE PATENTANMELDUNG

(11) **EP 0 958 794 A2**
(43) Veröffentlichungstag der Anmeldung: **24.11.1999**
(21) Anmeldenummer: 99107940.1
(22) Anmeldetag: 22.04.1999
(51) Int. Cl.: A61F 2/06

(54) **Radial aufweitbarer Stent zur Implantierung in ein Körpergefäss**

(30) Priorität: 16.05.1998 DE 19822157
(71) Anmelder: Jomed Implantate GmbH, 72414 Rangendingen (DE)
(72) Erfinder: von Oepen,Dr.-Ing.Randolf, 72145 Hirrlingen (DE)
(74) Vertreter: Schmitz, Hans-Werner, Dipl.-Ing.

(57) **Zusammenfassung**

Ein radial aufweitbarer Stent (10) zur Implantierung in ein Körpergefäß, insbesondere im Bereich einer Gefäßverzweigung, der mindestens einen Abschnitt (12) mit radialen Öffnungen (13) aufweist, deren Berandung (14) mindestens bereichsweise streifenförmig ausgebildet ist und eine oder mehrere Schleifen (15) und/oder Ausbuchtungen bildet.

## Beschreibung

Stents werden in der Regel nach einer Gefäßdilatation in die Gefäße eingesetzt und dort aufgeweitet, damit ein erneuter Verschluss des Gefäßes verhindert werden kann. Solche Gefäßverschlüsse können auch im Bereich von Gefäßverzweigungen auftreten, wobei gegebenenfalls der gesamte Verzweigungsbereich nach der Dehnung der Gefäße mit Stents gesichert werden muss. Hierzu ist bereits vorgeschlagen worden, in das Hauptgefäß einen ersten Stent einzuführen und anschließend durch eine radiale Öffnung dieses ersten Stents einen zweiten Stent in das abzweigende Gefäß einzuführen und anschließend ebenfalls aufzuweiten. Zur Ermöglichung des Hindurchführens des zweiten Stents durch eine radiale Öffnung des ersten Stents und zur Verhinderung eines zu großen Fließwiderstands im Verzweigungsbereich des Gefäßes für das Blut ist im DE-297 01 758 bereits ein Stent vorgeschlagen worden, der abschnittsweise vergrößerte radiale Öffnungen aufweist. In der Praxis hat sich jedoch gezeigt, dass diese Stents nicht überall einsetzbar sind. Im Bereich der vergrößerten radialen Öffnungen ist häufig die radiale Steifigkeit des Stents nicht ausreichend. Außerdem ist der Bedeckungsgrad der Gefäßwand im Bereich der vergrößerten Öffnungen zu gering, um ein Eindringen von sich ablösenden Gefäßablagerungen von der Gefäßwand in den Blutstrom zuverlässig zu verhindern. Diese abgelösten Ablagerungen können je nach Lage der Gefäße zu Embolien, Schlaganfällen und dergleichen führen.

Zur Abhilfe dieses Problems schlägt die vorliegende Erfindung einen radial aufweitbaren Stent zur Implantation in ein Körpergefäß, insbesondere im Bereich einer Gefäßverzweigung in Form eines hohlzylindrischen Elements vor, der dadurch gekennzeichnet ist, dass er mindestens einen Abschnitt mit radialen Öffnungen aufweist, deren Berandung mindestens bereichsweise streifenförmig ausgebildet ist und eine oder mehrere Schleifen und/oder Ausbuchtungen bildet. Die Schleifen und/oder Ausbuchtungen können dabei derart bemessen und angeordnet sein, dass der Durchmesser der radialen Öffnungen so vergrößerbar ist, dass ein zweiter, nicht aufgeweiteter Stent durch die radialen Öffnungen leicht hindurchführbar oder sogar im Bereich der Öffnungen radial aufweitbar ist. Dies ist dadurch möglich, dass die Schleifen oder Ausbuchtungen beim Hindurchführen beziehungsweise Aufweiten des zweiten Stents auseinandergezogen werden, wodurch sich die Querschnittsfläche der Öffnung stark vergrößern lässt. Diejenigen radialen Öffnungen, durch die kein zweiter Stent hindurchgeführt wird, weisen jedoch entweder die gleiche Querschnittsgröße wie radiale Öffnungen in anderen Abschnitten des Stents oder gegenüber diesen nur leicht vergrößerte Durchmesser auf. Dadurch ist sowohl eine ausreichende radiale Stabilität als auch ein ausreichender Bedeckungsgrad der Gefäßwand gewährleistet, um das Eindringen von sich von der Gefäßwand ablösenden Ablagerungen in den Blutfluss zuverlässig zu verhindern. Die radiale Steifigkeit des Stents in dem mindestens einen Abschnitt kann so eingestellt werden, dass sie mindestens annähernd der radialen Steifigkeit in den übrigen Abschnitten entspricht. Bei einer bevorzugten Ausführungsform ist der Stent über die Hälfte seiner Länge mit radialen Öffnungen versehen, deren Berandung mindestens bereichsweise streifenförmig ausgebildet ist und einen oder mehrere Schleifen und/oder Ausbuchtungen bildet. Die Platzierung des ersten Stents mit einer solchen Ausgestaltung ist dann relativ einfach, da er über eine relativ große Länge mit den speziell ausgestalteten radialen Öffnungen versehen ist. Der erfindungsgemäße Stent kann vorteilhafterweise aus einem massiven Röhrchen durch Laserschneiden oder dergleichen gefertigt werden. Weitere Vorteile ergeben sich, wenn er aus einem Material, das bei Röntgendurchstrahlung gut sichtbar ist, gefertigt oder mit einer Beschichtung aus einem solchen Material versehen ist. Als mögliche Materialien kommen beispielsweise Gold oder Platin in Frage.

Nachfolgend werden bevorzugte Ausführungsbeispiele eines erfindungsgemäßen Stents anhand der Zeichnung näher beschrieben.

Es zeigen:
- Fig. 1: eine Darstellung der Oberflächenstruktur eines erfindungsgemäßen Stents;
- Fig. 2: eine Darstellung der Oberflächenstruktur eines zweiten erfindungsgemäßen Stents.

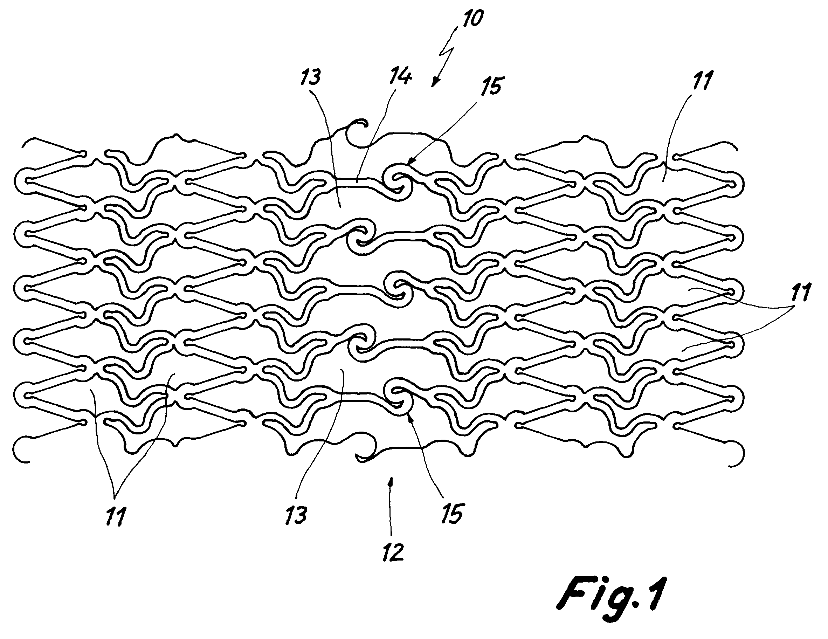

Fig. 1 zeigt einen Ausschnitt aus der Oberfläche eines Stents 10, der gleichmäßig über seine Oberfläche verteilt im Wesentlichen rautenförmige Öffnungen 11 aufweist. In einem mittleren Bereich 12 sind radiale Öffnungen 13 vorgesehen, deren Querschnittsfläche gegenüber der Querschnittsfläche der anderen radialen Öffnungen 11 nur leicht vergrößert ist. Die radialen Öffnungen 13 werden ebenso wie die radialen Öffnungen 11 von streifenförmigen Berandungen 14 umschlossen. Die Berandungen 14 der radialen Öffnungen 13 weisen dabei S-förmige Schleifen 15 auf. Dadurch ist es möglich, die Öffnungen 13 in ihrer Querschnittsfläche stark zu vergrößern. Der Stent 10 eignet sich somit ausgezeichnet zur Verlegung im Bereich von Gefäßverzweigungen. Durch eine der Öffnungen 13 kann mühelos durch Auffalten der S-förmigen Schleifen 15 ein zweiter Stent hindurchgeführt und radial gedehnt werden, sodass durch die Stents keine Beeinträchtigung des Blutflusses durch das Gefäß auftritt. Auch wenn das abzweigende Gefäß nicht mit einem Stent versehen werden muss, kann eine der radialen Öffnungen 13 im Durchmesser aufgeweitet werden, damit ein ungehinderter Blutfluss in das Nebengefäß möglich ist. Die Platzierung des Stents 10 im Gefäß gestaltet sich relativ einfach, da die Öffnungen 13 sich über den gesamten Stentumfang im Bereich 12 erstrecken.

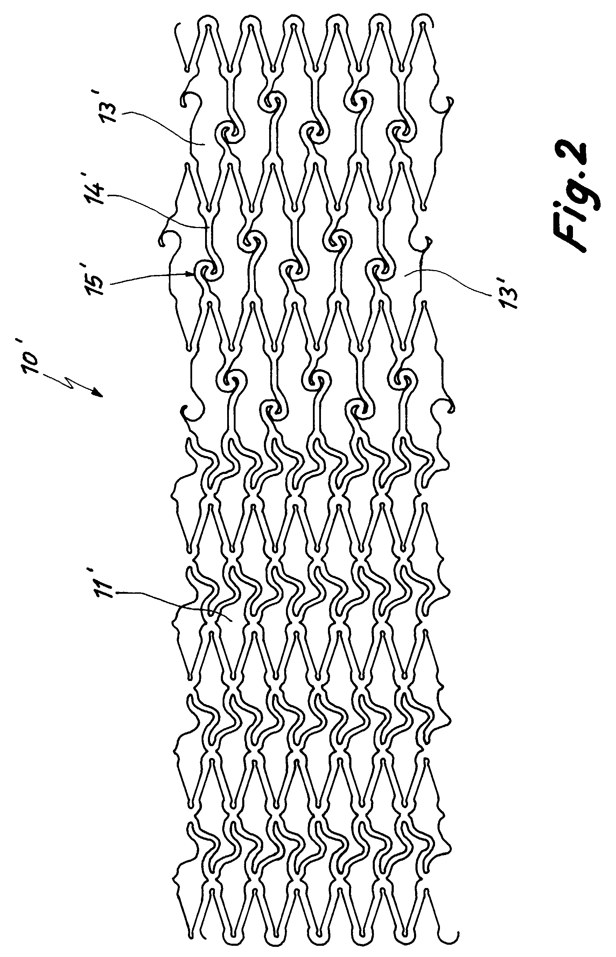

Im Gegensatz zum Stent 10 weist der Stent 10' aus Fig. 2 nicht nur in einem mittleren Bereich sondern über eine seiner Hälften verteilt radiale Öffnungen 13' mit Berandungen 14', in die S-förmige Schleifen 15' eingeformt sind, auf. Durch diese Ausgestaltung des Stents 10' ist die Platzierung innerhalb des Gefäßes in einem Verzweigungsbereich noch einfacher.

Beide Stents 10 und 10' haben den Vorteil, dass sie einen sehr guten Bedeckungsgrad der Gefäßwand ergeben und dennoch eine sehr starke Aufweitung einzelner radialer Öffnungen zum Hindurchführen eines zweiten Stents erlauben. Der hohe Bedeckungsgrad der Gefäßwand sorgt dafür, dass sich keine Ablagerungen an der Gefäßwand ablösen und in den Blutfluss gelangen können. Außerdem ist die radiale Steifigkeit der Stents 10 und 10' auch im Bereich der radialen Öffnungen 13' ebenso hoch wie im Bereich der radialen Öffnungen 11, 11'. Anstelle der S-förmigen Schleifen 15, 15' könnten natürlich auch meanderförmige Schleifen, Ausbuchtungen oder dergleichen in den Berandungen 14, 14' vorgesehen sein.

## Patentansprüche

1. Radial aufweitbarer Stent zur Implantierung in ein Körpergefäß, insbesondere im Bereich einer Gefäßverzweigung, in Form eines hohlzylindrischen Elements, dadurch gekennzeichnet, dass er mindestens einen Abschnitt (12) mit radialen Öffnungen (13, 13') aufweist, deren Berandung (14, 14') mindestens bereichsweise streifenförmig ausgebildet ist und eine oder mehrere Schleifen (15, 15') und/oder Ausbuchtungen bildet.

2. Stent nach Anspruch 1, dadurch gekennzeichnet, dass die Schleifen (15, 15') und/oder Ausbuchtungen derart bemessen und angeordnet sind, dass der Durchmesser der radialen Öffnungen (13, 13') so vergrößerbar ist, dass ein zweiter, nicht aufgeweiteter Stent durch die radialen Öffnungen (13, 13') leicht hindurchführbar ist.

3. Stent nach Anspruch 1 oder 2, dadurch gekennzeichnet, dass die Schleifen (15, 15') und/oder Ausbuchtungen derart bemessen und angeordnet sind, dass der Durchmesser der radialen Öffnungen (13, 13') 50 vergrößerbar ist, dass der zweite Stent auch im Bereich der Öffnungen (13, 13') radial aufweitbar ist.

4. Stent nach einem der Ansprüche 1 bis 3, dadurch gekennzeichnet, dass der mindestens eine Abschnitt (12) hohlzylindrisch ist.

5. Stent nach einem der Ansprüche 1 bis 4, dadurch gekennzeichnet, dass der Durchmesser der radialen Öffnungen (13, 13') in dem mindestens einen Abschnitt (12) gegenüber dem Durchmesser radialer Öffnungen (11, 11') in anderen Abschnitten des Stents (10, 10') leicht vergrößert sind.

6. Stent nach einem der Ansprüche 1 bis 5, dadurch gekennzeichnet, dass seine radiale Steifigkeit in dem mindestens einen Abschnitt (12) wenigstens annähernd der radialen Steifigkeit in den übrigen Abschnitten entspricht.

7. Stent nach einem der Ansprüche 1 bis 6, dadurch gekennzeichnet, dass er über die Hälfte seiner Länge radiale Öffnungen (13') aufweist, deren Berandung (14') mindestens bereichsweise streifenförmig ausgebildet ist und eine oder mehrere Schleifen (15') und/oder Ausbuchtungen bildet.
